# EUROPÄISCHE PATENTSCHRIFT

(11) **EP 1 750 574 B1**
(45) Veröffentlichungstag und Bekanntmachung des Hinweises auf die Patenterteilung: **12.11.2014**
(21) Anmeldenummer: 05739588.1
(22) Anmeldetag: 12.05.2005
(51) Int. Cl.: A61B 5/00

(54) **MEDIZINISCHE KAMERA**
MEDICAL CAMERA
CAMERA MEDICALE

(30) Priorität: 16.05.2004 DE 102004024494
(43) Veröffentlichungstag der Anmeldung: 14.02.2007
(60) Teilanmeldung: 11002027.8
(73) Patentinhaber: Dürr Dental AG, 74321 Bietigheim-Bissingen (DE)
(72) Erfinder: THOMS, Michael, 74321 Bietigheim-Bissingen (DE); LAIS, Peter, 74391 Erligheim (DE)
(74) Vertreter: Ostertag, Reinhard
(86) Internationale Anmeldenummer: PCT/EP2005/005133
(87) Internationale Veröffentlichungsnummer: WO 2005/110206

(56) Entgegenhaltungen:
- EP-A- 1 099 405
- WO-A-01/26576
- US-B1- 6 537 211
- US-B1- 6 697 657
- US-B1- 6 697 666

## Beschreibung

Die Erfindung betrifft eine medizinische Kamera.

Eine derartige, als Endoskop ausgebildete Kamera ist der EP 1 099 405 A1 zu entnehmen. Diese umfasst eine Betriebseinheit, welche Lichtquelle und Elektronik umfasst, sowie ein lösbar mit dieser verbundenes Endoskop. Das Endoskop umfasst einen Lichtleiter, der sich von einem Licht-Austrittsfenster zur Lichtquelle der Betriebseinheit erstreckt. Das Endoskop hat ferner einen Bildwandler, auf welchen von einem Untersuchungsbereich zurücklaufendes Fluoreszenzlicht über ein Ein-trittsfenster des Endoskopes und ein Objektiv abgebildet wird. Das Spektrum des von der Betriebseinheit abgegebenen Anregungslichtes lässt sich über eine umlaufende Filterscheibe zwischen den Farben rot, grün und blau umstellen.

In der US 6 697 657 B1 ist ein Endoskop beschrieben, welches zur Fluoreszenzdiagnose von inneren Organen bestimmt ist. Ein Kathederteil ist über einen dichroitischen Spiegel einerseits an eine Laser-Lichtquelle und andererseit an einen photoelektrischen Sensor angekoppelt. Auf diese Weise kann man denselben Lichtleiter dazu benutzen, Anregungslicht an den Untersuchungsbereich zu überstellen und dort induziertes Fluoreszenzlicht auf den Sensor zurückzuführen.

Die US 6 697 666 B1 betrifft eine Kamera zum Diagnostizieren von Geweben, z. B. von Geweben des Cervikalkanals. Als Anregungslichtquelle wird eine Xenolampe mit nachgeschaltetem Filterrad verwendet. Eine mit dem Fluoreszenzlicht beaufschlagte Detektionseinrichtung umfasst ein Spektrometer in Kombination mit einem CCD-Array. Damit kann die spektrale Verteilung von einem Untersuchungsbereich zurücklaufenden Fluoreszenzlichtes ermittelt werden.

Die US 6 637 211 B1 offenbart ein Endoskop mit einer Laserlichtquelle für UV-Licht und einer Weißlichtquelle. Das Licht dieser beiden Quellen wird comutergesteuert durch getaktete Verschlüsse zerhackt. Das Fluoreszenzlicht wird unter Verwendung von Kantenfiltern analysiert. Normalerweise kann eine Xenon-Bogenentladungslampe oder eine Quecksilber-Bogenentladungslampe als Lichtquelle verwendet werden.

Die WO 01/26576 A1 zeigt eine UV-Lichtquelle zur Verwendung bei photochemischen Reaktionen im dentalen Bereich. Diese Lichtquellen können eine Mehrzahl von LEDs aufweisen.

Medizinische Kameras mit einem elektronischen

Bildwandler finden in der Medizin zunehmend Verbreitung, um medizinische Befunde rasch darstellen zu können, die Bilder einer Bildbearbeitung unterwerfen zu können und so mehr Information zu erhalten oder und um die Bilder preisgünstig und rasch archivieren zu können.

Es ist bekannt, daß eine Vielzahl von Bakterien unter optischer Anregung mit Licht eine Fluoreszenz zeigt, welche als qualitatives oder gar quantitatives Merkmal einer Erkrankung dienen kann. Die spektralen Eigenschaften der Fluoreszenz werden dabei von bestimmten Molekülen, die in den Bakterien vorkommen und z.B. beim Stoffwechsel der Bakterien entstehen, bestimmt. So entstehen in vielen Bakterien als Stoffwechselprodukte Porphyrine, welche Absorptionsbanden im Bereich von 350 - 450 nm (Soret-Band) sowie von 500 - 640 nm (Q-Banden) und Emissionsbanden bei 630 ± 50 nm besitzen.

Zum Nachweis durch solche Bakterien verursachter Erkrankungen wird auf den erkrankten Bereich mit Licht einer Längenwelle eingestrahlt, welche zu einem jeweiligen Fluoreszenzmolekül des Bakteriums paßt. Das vom Bakterium emittierte Licht wird mit einem Detektor, welcher nur auf die Lichtsignale bei der entsprechenden Emissionslängenwelle reagiert, nachgewiesen.

Durch die vorliegende Erfindung soll eine medizinische Kamera der eingangs angesprochenen Art geschaffen werden, welche kompakten Aufbau aufweist und sich insbesondere zur Untersuchung von Gewebeoberflächen in Körperhöhlen, z.B. im Mund, eignet.

Diese Aufgabe ist erfindungsgemäß gelöst durch eine medizinische Kamera mit dem im Anspruch 1 angegebenen Merkmalen.

Bei der erfindungsgemäßen Kamera ist sichergestellt, daß anregendes UV-Licht nicht direkt das Eintrittsfenster erreichen kann. Auf diese Weise ist der durch Anregungslicht bedingte Untergrund im erhaltenen Bild klein. Daher wird auch schwaches Fluoreszenzlicht gut sichtbar dargestellt.

Vorteilhafte Weiterbildungen der Erfindung sind in Unteransprüchen angegeben.

Mit der Weiterbildung der Erfindung gemäß Anspruch 2 wird erreicht, daß das UV-Licht vom Bildwandler fern gehalten wird, das Fluoreszenzlicht dagegen in allen Anteilen für die Bilderzeugung genutzt wird.

Kantenfilter, wie sie in den Ansprüchen 3 und 4 angegeben sind, eignen sich besonders gut für medizinische Zwecke.

Bei einer Kamera gemäß Anspruch 5 erzielt man eine hohe Intensität des UV-Lichtes bei geringer Wärmeentwicklung. Letztere wird vom Patienten als unangenehm empfunden und könnte auch zu Gewebebeschädigungen führen.

Mit der Weiterbildung der Erfindung gemäß Anspruch 6 wird eine gleichförmige Ausleuchtung des Gesichtsfeldes erhalten.

Die Weiterbildung der Erfindung gemäß Anspruch 7 gestattet es, eine Beobachtungsstelle intensiv mit UV-Licht zu bestrahlen und eine entsprechend hohe Intensität des Fluoreszenzlichtes zu erhalten. Dabei ist aber insgesamt über das Tastverhältnis des Betriebs der UV-Lichtquelle gewährleistet, daß keine Gewebebeschädigungen entstehen.

Bei einer Kamera gemäß Anspruch 8 kann der Benutzer die Dauer und den Abstand der einzelnen von der UV-Lichtquelle abgegebenen Lichtimpulse nach Bedarf einstellen.

Eine Kamera gemäß Anspruch 9 gestattet es, den zu untersuchenden Gewebebereich auch mit Weißlicht zu betrachten, was oft zur Ergänzung der Fluoreszenzbetrachtung gewünscht wird.

Auch die Weiterbildung der Erfindung gemäß Anspruch 10 dient der gleichförmigen Ausleuchtung des Gesichtsfeldes.

Bei einer Kamera gemäß Anspruch 11 erhält man ein besonders kontrastreiches Bild erkrankter Gewebestellen, da das Untergrundsbildagezogen wird.

Eine Kamera gemäß Anspsruch 12 gestattet es, den zu untersuchenden Gewebebereich mit UV-Licht unterschiedlicher Wellenlänge zu beleuchten. Hierdurch erhält man eine zusätzliche Möglichkeit der Unterscheidung von gesundem und krankem Gewebe bzw. unterschiedlich erkrankten Gewebebereichen.

Dabei ist wieder die Maßnahme gemäß Anspruch 13 im Hinblick auf ein besonders kontrastreiches Bild von Vorteil.

Die Weiterbildung der Erfindung gemäß Anspruch 14 ist im Hinblick auf die Untersuchung von schlecht zugänglichen Gewebebereichen von Vorteil.

Mit der Maßnahme gemäß Anspruch 15 wird erreicht, daß der zu untersuchende Bereich von Verunreinigungen wie Blut freigehalten wird.

Bei handelsüblichen Kameras ist die Optik wie generell bei Kameras als telezentrische Optik aufgebaut. Um bei einer solchen Optik gute Abbildungseigenschaften zu gewährleisten, müssen die objektseitige Linsenanordnung, die mittlere Linsenanordnung und die wandlerseitige Linsenanordnung aus mehreren (in der Regel zwei) Einzellinsen aufgebaut werden. Aus diesem Grunde sind die Optiken für derartige Kameras teuer.

Eine Kamera gemäß Anspruch 16 kann bei weiterhin guten Abbildungseigenschaften preiswerter hergestellt werden kann.

Bei nicht telezentrischem Strahlengang, kann man bei medizinsichen Kameras auch Linsenanordnungen verwenden, die jeweils für sich genommen keine besonders guten Abbildungseigenschaften aufweisen. Insbesondere kann man die einzelnen Linsenanordnungen auch durch einzelne Linsen realisieren. Hierdurch wird eine beträchtliche Kostenersparnis erhalten, da weniger Linsen benötigt werden und auch die Montage der Optik vereinfacht ist.

Legt man die Gesichtsfeldblende so, wie im Anspruch 17 angegeben, so hat man besonders gute Abbildungseigenschaften. Die dritte Linsenanordnung wird in ihrem mittleren Bereich genutzt, wo die Verzeichnungsfehler klein sind. Die mittlere Linsenanordnung wird auch in ihren Randbereichen genutzt. Sie braucht aber keine stark gekrümmten Oberflächen aufzuweisen, so dass auch die Nutzung der Randbereiche dieser Linsenanordnung nicht zu nicht akzeptablen Verzeichnungsfehlern führt.

Gemäß Anspruch 18 kann man die erste Linsenanordnung aus nur einer optischen Komponente aufbauen, die sehr einfache Geometrie aufweist.

Auch die Weiterbildungen der Erfindung gemäß den Ansprüchen 19 und 20 geben besonders einfache Möglichkeiten an, die mittlere Linsenanordnung bzw. die wandlerseitige Linsenanordnung zu realisieren.

Eine Kamera, wie sie in Anspruch 21 angegeben ist, eignet sich besonders gut zur Verwendung für dentale Zwecke, da die Betrachtungsrichtung zur Achse des Handstückes geneigt ist, insbesondere senkrecht auf dieser steht.

Die Weiterbildung der Erfindung gemäß Anspruch 22 ist im Hinblick auf ein einfaches Reinigen und Sterilisieren der Kamera von Vorteil.

Eine Kamera gemäß Anspruch 23 kann sowohl zur Betrachtung eines Gegenstandes aus unmittelbarer Nähe als auch zur Betrachtung eines Gegenstandes aus größerer Entfernung verwendet werden. Bei einer dentalen Kamera kann der Arzt zum Beispiel Einzelheiten eines Zahnes oder einen Gesamtüberblick über das Gebiss aufnehmen.

Anspruch 24 gibt eines besonders einfache Möglichkeit der Einstellung des Aufnahmeabstandes.

Bei einer Kamera gemäß Anspruch 25 ist die Einstellung des Aufnahmeabstandes möglich, ohne dass ein bewegtes Teil durch die Wand des Kameragehäuses hindurchgeführt werden muss.

Nachstehend wird die Erfindung anhand von Ausführungsbeispielen unter Bezugnahme auf die Zeichnung näher erläutert. In dieser zeigen:
- Figur 1:: eine axialen Schnitt durch eine dentale Kamera;
- Figur 2:: eine schematische Ansicht der Optik der dentalen Kamera nach Figur 1;
- Figur 3:: eine ähnliche Ansicht wie Figur 1, in welcher jedoch eine abgewandelte Verstellmechanik für den Bildwandler gezeigt ist;
- Fig. 4:: eine Aufsicht auf den Kopf einer Diagnosekamera sowie eine schematische Darstellung der mit dem Bildwandler der Kamera zusammen arbeitenden Betriebseinheit; und
- Fig. 5:: eine schematische Darstellung einer abgewandelten Kamera, die an optisch schwer zugänglichen Stellen verwendet wird.

Die in der Zeichnung wiedergegebene dentale Kamera hat ein Gehäuse 10, das als Plastikspritzteil hergestellt ist. Das Gehäuse 10 ist als einstückiges Gehäuse wiedergegeben; es versteht sich, dass der Fachmann es je nach den Herstellungserfordernissen als mehrteiliges Gehäuse konstruieren kann, wobei die verschiedenen Gehäuseteile dann unter Zwischenschaltung von Dichtungen dicht miteinander verbunden werden oder miteinander verklebt oder verschweißt werden.

Das Gehäuse 10 hat einen Griffabschnitt 12, der im wesentlichen die Form einer an den Enden geschlossenen zylindrischen Hülse hat. Der Griffabschnitt 12 trägt an seinem freien Ende einen sich verjüngenden und abgewinkelten Gehäuseabschnitt 14, dessen nach unten gewandtes Ende durch ein Eintritts-Fenster 16 und ein danebenliegendes Lichtaustritts-Fenster 17 bündig und dicht verschlossen ist.

Das Eintrittsfenster 16 ist zugleich als Kantenfilter ausgebildet. Es kann sich hierbei um ein Farbglasfilter mit einer Kante bei etwa 550 nm handeln, wie es von der ITOS-Gesellschaft für technische Optik mbH unter der Bezeichnung OG 550 vertrieben wird. Ein Farbglasfilter mit einer näher bei der Wellenlänge des UV-Lichtes liegenden Kante ist z.B. das Filter GG 495 der Firma Schott.

In dem Gehäuse 10 ist eine insgesamt mit 4 bezeichnete Optik angeordnet, die ein schematisch angedeutetet Objekt 6 (Zahn oder Kieferbogen) auf einen Bildwandler 8 abbildet. Bei dem Bildwandler 8 kann es sich um ein Farb-CCD handeln.

In dem abgewinkelten Teil des Gehäuseabschnittes 14 ist ein Umlenkspiegel 18 angeordnet, der unter 45 Grad zur Achse des Griffabschnittes 12 und zur Achse des Fensters 16 angestellt ist und auch als Umlenkprisma, beispielsweise als Rechtwinkel- oder Pentaprisma, ausgebildet sein kann.

Im Strahlengang hinter dem Umlenkspiegel 28 ist eine Linse 22 angeordnet, die eine konkave vordere Stirnfläche 24 und eine konvexe hintere Stirnfläche 26 aufweist.

Unter größerem Abstand von der Linse 22 ist eine Zwischenlinse 28 angeordnet, welche eine konvexe objektseitige Stirnfläche 30 und eine konvexe wandlerseitige Stirnfläche 32 aufweist.

Wiederum eine größere Strecke hinter der Zwischenlinse 24 ist eine wandlerseitige Linse 34 vorgesehen, die eine konvexe objektseitige Stirnfläche 36 und eine konvexe wandlerseitige Stirnfläche 38 aufweist.

Der Bildwandler 8 ist auf einem Schlitten 40 angeordnet, der durch auf der Innenseite des Gehäuses 10 vorgesehene Führungsrippen 42, 44 längs der Achse der Optik 4 bewegbar geführt ist. Auf der einen Längsfläche des Schlittens 40 ist eine Zahnstange 46 ausgebildet, die mit einem Zahnrad 48 kämmt, welches drehbar am Gehäuse 10 gelagert ist und mit einem Zahnradabschnitt durch das Gehäuse 10 nach außen übersteht. Durch Drehen des Zahnrades 48 kann somit der Bildwandler 8 längs der Achse der Optik 4 verstellt werden.

In dem Gehäuse 10 ist ein im wesentlichen in axialer Richtung verlaufender Kanal 50 vorgesehen, in welchem ein Lichtleiter 52 vorgesehen ist.

Hinter dem vom Fenster 17 abgelegenen Ende des Lichtleiters 52 sitzt eine UV-LED 55, die ultraviolettes Licht mit einer Wellenlänge zwischen 390 und 410 nm emittiert. Derartige UV-LEDs werden z.B. von der Firma ETG unter der Typ-Bezeichung ETG-3UV400-30 vertrieben. Das Halbleitermaterial ist ein InGaN, welches im blauen UV emittiert. In die LED ist eine Linse integriert, so daß man insgesamt ein sehr schmales Lichtbündel erhält.

Ein Endabschnitt des Kanales 50 und des Lichtleiters 52 sind so abgewinkelt, dass auf den Lichtleiter 52 gegebenes Licht den Lichtleiter 52 leicht geneigt zur Achse des Fensters 17 verlässt, wie bei 54 gezeigt.

Der Bildwandler 8 und der Lichtleiter 52 sind über eine in der Zeichnung nicht wiedergegebene (dort rechts zu denkende) Steckverbindung mit einer Bildauswerteelektronik verbunden.

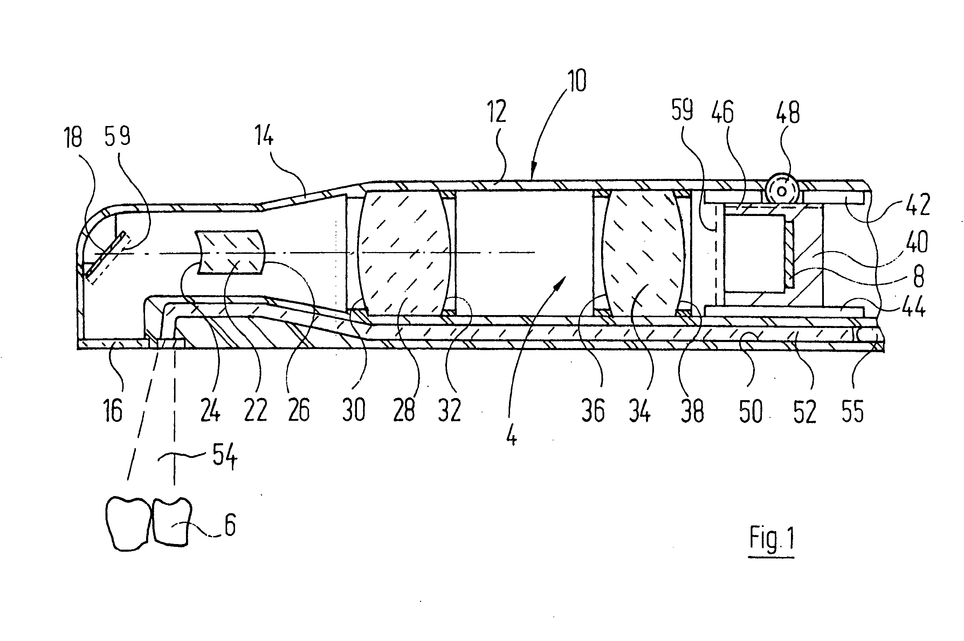

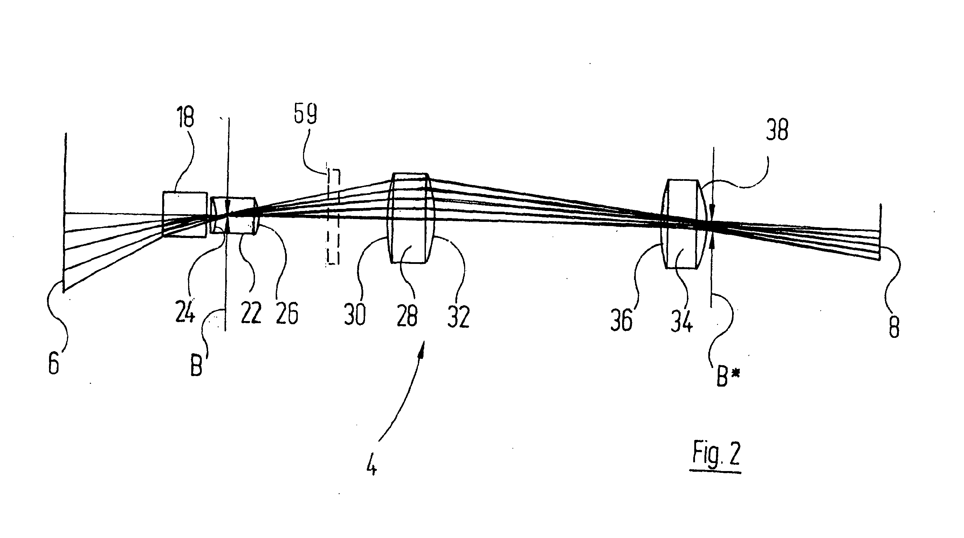

Der Strahlengang der Optik 4 ist in Figur 2 nochmals genauer gezeigt. Der besseren Darstellbarkeit halber wurden die Verhältnisse so gezeigt, wie sie bei einer Geradsicht-Kamera bestehen würde, die man aus der Kamera nach Figur 1 erhält, wenn man den als Umlenkprisma ausgebildeten Umlenkspiegel 18 durch eine planparallele Glasplatte gleicher optischer Dicke ersetzt und das Fenster 16 auf der Achse des Griffabschnittes 12 vorsieht. Die verschiedenen optischen Komponenten sind wieder so bezeichnet wie in Figur 1. Zusätzlich sind verschiedene Strahlen eingezeichnet, die von unterschiedlichen Punkten des Objektes 6 zu zugeordneten Punkten auf der Oberfläche des Bildwandlers 8 führen.

Man erkennt, dass bei der in Figur 2 gezeigten Optik der Gesichtsfeldblende B ein Bild B konjugiert ist, welches in der Nähe der Linse 22 angeordnet ist. Man erkennt, dass bei solcher Lage der Gesichtsfeldblende B bzw. des Bildes B derselben die objektseitige Linse 22 im wesentlichen in ihrem mittleren Bereich genutzt wird, die Zwischenlinse 28 auch in ihren Randbereichen genutzt wird und die wandlerseitige Linse 34 wieder nur in ihrem zentralen Bereich genutzt wird.

Aufgrund der gezeigten Anordnung der drei Linsen, bei welcher die Zwischenlinse 28 sowohl von der objektseitigen Linse 22 als auch von der wandlerseitigen Linse 34 deutlich beabstandet ist, braucht die Zwischenlinse 28 keine scharf gekrümmten Oberflächen zu haben. Hierdurch werden optische Aberrationen reduziert. Die Tatsache, dass in der Zwischenlinse 28 auch die Randbereiche genutzt werden, führt somit nicht zu einer nicht hinnehmbaren Verzeichnung des Bildes.

Die nachstehende Tabelle gibt ein konkretes Ausführungsbeispiel für eine Möglichkeit der Realisierung der Optik 4 an. Die Verhältnisse entsprechen der Darstellung von Figur 2.

Dabei ist jeweils aufgeführt die Nummer der Stirnfläche (Bezugszeichen von Figur 1 bzw. 2), der Krümmungsradius der entsprechenden Stirnfläche, die Dicke der Materialschicht, die sich an die Stirnfläche anschließt, und die Art des optischen Mediums (Glasart; L = Luft), welches hinter der entsprechenden Fläche liegt. In der letzten Spalte ist der Durchmesser der jeweiligen Stirnfläche angegeben. Längeneinheit ist jeweils 1 mm.

| Fläche | Radius | Dicke | Glas | Durchmesser |
|---|---|---|---|---|
| Objekt | ∞ | 9 | L | 13,21 |
| 19 | ∞ | 4 | SF8 | 3,80 |
| 21 | ∞ | 0,76 | | 1,54 |
| 24 | -2,31 | 4,00 | N-LASF30 | 3,00 |
| 26 | -2,65 | 11,83 | L | 3,00 |
| 30 | 24,30 | 4 | N-LASF30 | 7,50 |
| 32 | -11,94 | 20,84 | L | 7,50 |
| 36 | 12,15 | 4,00 | N-ZK7 | 7,50 |
| 38 | -8,82 | 0,56 | L | 7,50 |
| Blende B***** | ∞ | 15,13 | L | 0,98 |
| Wandler | ∞ | | | 4,85 |

Soweit in der Spalte Glas "L" angegeben ist, handelt es sich um Luftstrecken. Die Glastypen entsprechen dem Katalog für optische Gläser der Firma Schott.

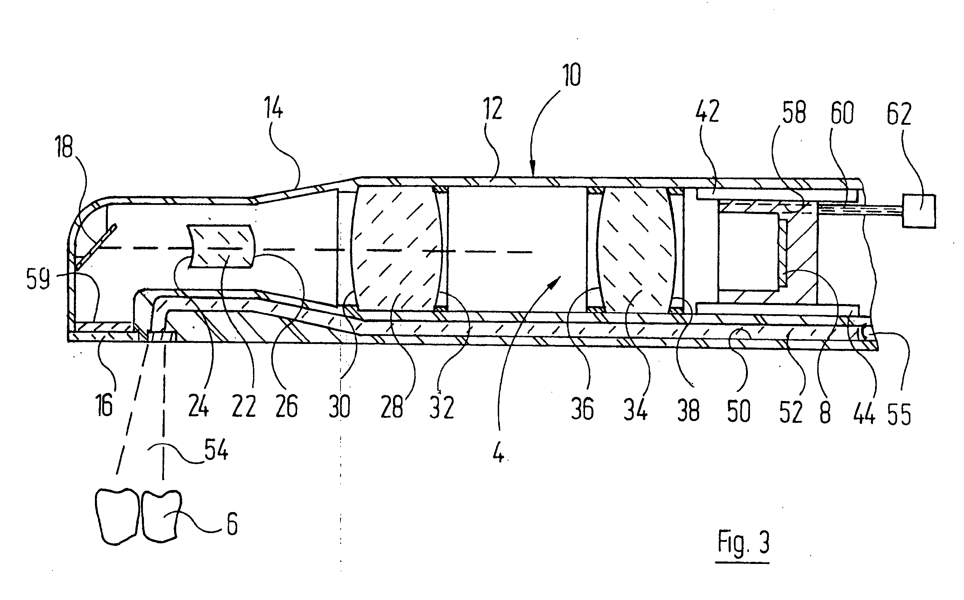

Das Ausführungsbeispiel nach Figur 3 entspricht weitgehend demjenigen nach Figur 1; entsprechende Komponenten sind wieder mit denselben Bezugszeichen versehen und werden nicht nochmals im einzelnen beschrieben.

Beim Ausführungsbeispiel nach Figur 3 ist der Schlitten 40 mit einer Gewindebohrung 58 versehen, in welcher eine Gewindelspindel 60 läuft. Die Gewindespindel 60 wird von einem Elektromotor 62 angetrieben, der vom Gehäuse 10 getragen ist. Die Versorgungsleitungen für den Elektromotor 62 gehen genauso über die rechts von Figur 3 zu denkende Steckverbindung zu einem Versorgungsschlauch wie die Anschlussleitungen des Bildwandlers 8 und der Lichtleiter 52.

Auf diese Weise kann der Wandler 8 längs der Achse der Optik 4 verstellt werden, ohne dass eine mechanische Durchführung durch die Wand des Gehäuses 10 vorgesehen werden müsste.

In Abwandlung der oben beschriebenen Ausführungsbeispiele kann man das Eintrittsfenster 16 auch als vollständig transparentes Fenster ausbilden und ein zusätzliches Farbfilter 59 auf den Umlenkspiegel 18 setzen, was den Vorteil hat, daß das Filter zweimal vom Beobachtungslicht durchsetzt wird. Nochmals alternativ kann man auch das Farbfilter 59 vor den Schlitten 50 setzen, wie gestrichelt dargestellt, oder direkt über den Bildwandler 8 legen.

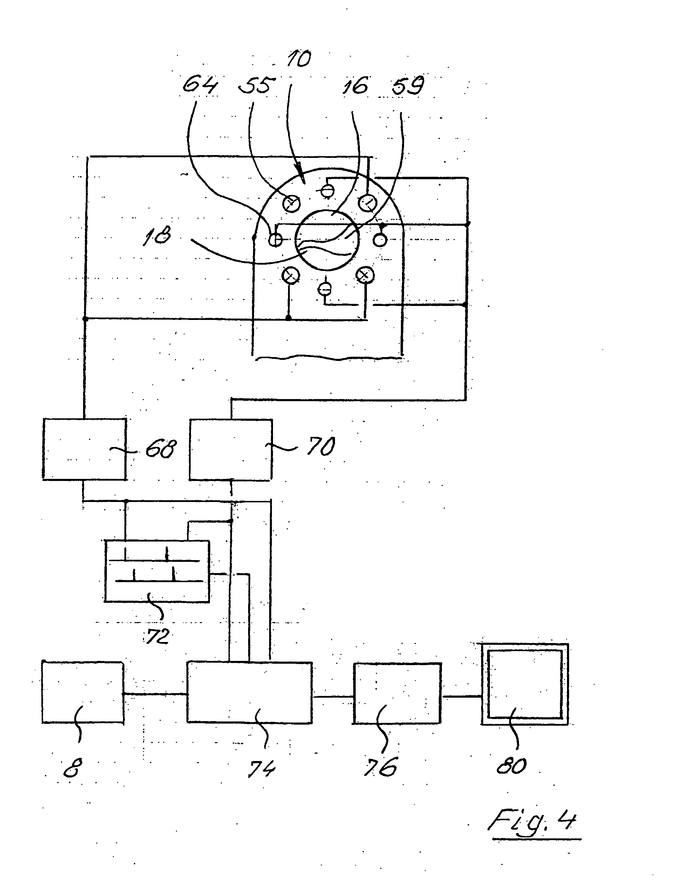

Beim Ausführungsbeispiel nach Fig. 4 sind Bauelemente, die obenstehend unter Bezugnahme auf die Fig. 1 bis 3 schon erläutert wurden, wieder mit denselben Bezugszeichen versehen. Sie brauchen nachstehend nicht nochmals detailliert beschrieben werden.

Um das kreisförmige Eintrittsfenster 16 herum sind in Umfangsrichtung gleich verteilt vier Weißlicht-LEDs 64 verteilt. Zwischen diesen liegen ebenfalls in Umfangsrichtung gleich verteilt vier UV-LEDs 66.

Die Weißlicht-LEDs 64 sind mit dem Ausgang einer Betriebsschaltung 68 verbunden, welche die Weißlicht-LEDs 64 jeweils nach Wahl durchgehend oder für Zeitspannen einschaltet.

Ähnlich sind die UV-LEDs 55 mit einer Betriebsschaltung 70 verbunden, welche die UV-LEDs jeweils für kurze Zeitspannen aktiviert.

Die Steuerung der Betriebsschaltung 70 erfolgt durch einen Taktgeber 72, der neben erste und zweiten Aktivierungsimpulsen für die Betriebsschaltung 70 und ggf. 68 (falls getaktet, was hier angenommen wird) noch gegenüber diesen beiden phasenverschobene Steuerimpulse bereitstellt. Diese werden somit zu Zeitpunkten erzeugt, in denen die UV-LEDs (und ggf. die Weißlicht-LEDs, falls getaktet) nicht arbeiten.

Mit den beiden Taktimpulsen und den Steuerimpulsen des Taktgebers 72 ist ein Rechenkreis 74 beaufschlagt. Dieser ist an seinem Eingang mit dem Ausgang des Bildwandlers 8 verbunden.

Jedesmal dann, wenn der Taktgeber 72 einen ersten Aktivierungsimpuls erhält, lädt er aus einem mit ihm verbundenen Bildspeicher 76 das bisher auf integrierte Fluoreszenz-Bild und zählt amplitudenmäßig das gerade vom Bildwandler 8 geladene Bild hinzu. Dann speichert er das so erhaltene Gesamtbild wieder in den Bildspeicher 76 zurück.

Jedesmal dann, wenn der Taktgeber 72 einen zweiten Aktivierungsimpuls erhält, lädt er aus einem mit ihm verbundenen Bildspeicher 76 das bisher auf integrierte Weißlicht-Bild und zählt amplitudenmäßig das gerade vom Bildwandler 8 geladene Bild hinzu. Dann speichert er das so erhaltene Gesamtbild wieder in den Bildspeicher 76 zurück.

Erhält der Rechenkreis 74 einen Steuerimpuls, so lädt er ebenfalls den Inhalt des Bildspeichers 76 (Fluoreszenz-Bild und Weißlicht-Bild) und zieht von diesem das vom Bildwandler 8 erhaltene Bild amplitudenmäßig ab und speichert das so erhaltene neue Gesamtbild wieder in den Bildspeicher 76 zurück.

Man erkennt, daß auf diese Weise der Bildspeicher 76 ein Fluoreszenz-Bild enthält, welches nur die von Bakterien erzeugte Fluoreszenz zeigt, bei der jedoch das durch Umgebungslicht erzeugte Hintergrundsbild abgezogen ist.

Gleiches gilt für das Weißlicht-Bild.

Der Inhalt des Bildspeichers 76 kann auf einem Bildschirm 78 dargestellt werden.

In Abwandlung des Ausführungsbeispiels nach Fig. 4 kann man die Weißlicht-LEDs 64 auch durch weitere UV-LEDs ersetzen, die bei einer anderen Wellenlänge arbeiten als die UV-LEDs 66. Auch kann man sowohl Weißlichtdioden als auch mehrere Sätze bei unterschiedlichen Wellenlängen arbeitender UV-LEDs vorsehen. Die Ansteuerung der verschiedenen LEDs wie oben beschrieben.

Dabei erfolgt für die weiteren Sätze von UV-LEDs das Abziehen des Hintergrundsbildes genauso wie oben beschrieben.

Zusätzlich kann man die mit den verschiedenen Sätzen von UV-LEDs erhaltenen Bilder additiv oder subtraktiv zusammensetzen, um zusätzliche Strukturen in den Kranken Geweben zu erkennen.

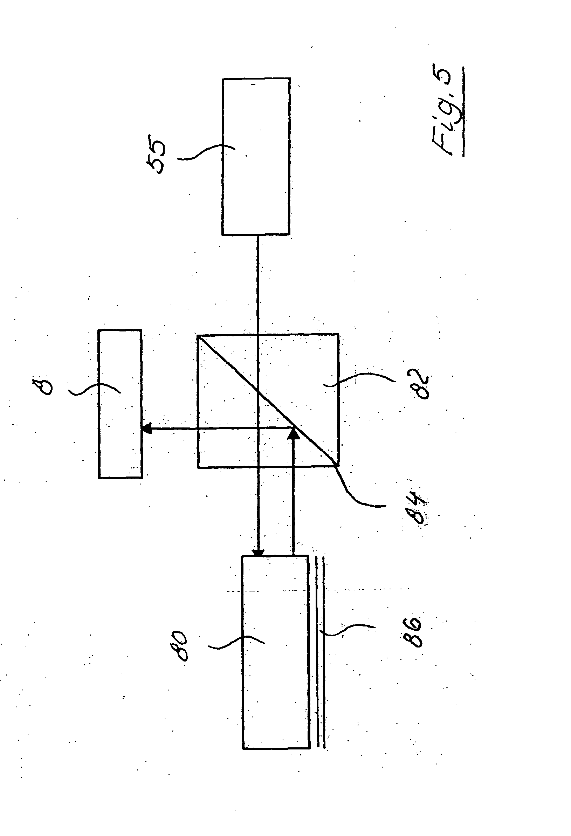

Beim Ausführungsbeispiel nach Fig. 5 sind Bauelemente, die obenstehend schon beschrieben wurden, wieder mit denselben Bezugszeichen versehen.

Die Kamera ist nur in ihren wichtigsten Bestandteilen gezeigt. Zum Beobachten sehr schlecht zugänglicher BEreiche, wie eines tiefen Spaltes, wie den Nasennebenhöhlen, dem Ohr oder einem Zahnwurzelspalt, findet anstelle einer Linsenoptik eine Faseroptik Verwendung. Diese ist schematisch bei 80 wiedergegeben.

Die UV-LED 55 beleuchtet das hintere Ende der Faseroptik 80 über einen dichroitischen Strahlteiler 82 mit einer wellenlängendispersiven Schicht 84, und das durch die Faseroptik 80 zurückkehrende Beobachtungslicht wird über den Strahlteiler 82 auf einen Bildwandler 8 gegeben, der nur noch ein Pixel zum Erfassen des Lichtes braucht, z.B. durch eine lichtempfindliche Diode oder einen Fototransistor gebildet sein kann.

Bei der in Fig. 5 gezeigten Kamera wird das UV-Licht durch durch die für sie durchlässige Schicht 84 und die Faseroptik 80 zu dem zu untersuchenden Bereich geleitet. Das zurücklaufende Beobachtungslicht läuft durch die Faseroptik 80 zurück und gelangt über die dichroitische Schicht 84 auf den Bildwandler 8.

Die Faseroptik 80 kann, falls gewünscht, zwei getrennte Teilbündel umfassen, von denen das eine das zum zu untersuchenden Bereich laufende UV-Licht und das andere das von dem zu untersuchenden Bereich zurücklaufende Beobachtungslicht leitet.

Im Falle von Verunreinigungen an der Untersuchungsstelle, z.B. im Wurzelspalt, kann es zweckmäßig sein, den zu untersuchenden Bereich von Verschmutzungen wie Bluzt freizuhalten. Man kann dann parallel zur Faseroptik 80 eine Hohlfaser 86 vorsehen, durch welche eine Spülflüssigkeit zum zu untersuchenden Bereich bepumpt wird.

In weiterer Abwandlung kann man auch die Faseroptik 80 selbst so auslegen, daß sie zugleich als Fluidkanal dient, durch welchen eine Spülflüssigkeit zum zu untersuchenden Bereich geleitet wird.

Das Einspeisen der Flüssigkeit erfolgt dann durch eine transversale Bohrung am Einkoppelende der Faseroptik.

Die oben beschriebene Kamera ist aufgrund ihrer hohen Empfindlichkeit dazu geeignet, die Eigenfluoreszenz von Bakterien nutzend gesunde und kranke Gewebebereiche zu unterscheiden. Es versteht sich, daß man diese Kamera aber auch dann verwenden kann, wenn die Bakterien durch einen zusätzlich applizierten Fluoreszenzmarker markiert worden sind. Solche Marker werden vorzugsweise vor der Untersuchung in flüssiger Lösung dem zu untersuchenden Bereich zugeführt und reichern sich spezifisch an den zu untersuchenden Bakterien an. Verwendet man eine Fasersensor-Kamera, kann der Fluoreszenzmarker vor der Untersuchung durch deren Fluidkanal (Hohlräume der Faseroptik oder zusätzliche Hohlfaser) zugeführt werden. Folgende weitere Abwandlungen der Erfindung sind möglich:

Da die Fluoreszenz in der Intensität sehr schwach sein kann und weiterhin breitbandiges Störlicht z.B. durch andere fluoreszierende Zellen oder Umgebungslicht vorhanden sein kann, kann es zweckmäßig sein, mit mehreren Photodetektoren, welche bei unterschiedlichen Wellenlängen empfindlich sind, sowohl die Intensität des Störsignals als auch die Intensität des Fluoreszenzsignals, welches mit dem Störsignal ebenfalls überlagert ist, zu messen und durch Differenzbildung das Fluoreszenzsignal zu gewinnen.

Eine andere Möglichkeit, ein Störsignal durch Umgebungslicht zu unterdrücken, kann darin bestehen, das Anregungslicht zu pulsen und während dieser Zeit und der Abklingzeit des Fluoreszenzsignals die abgestrahlte Intensität zu integrieren und während eines späteren vorzugsweise gleich lang dauernden Zeitintervalls die Störlichtintensität zu integrieren, so daß ebenfalls aus der Differenz der beiden Signale das Fluoreszenzsignal gewonnen wird.

Beispielsweise wird mit einer ultravioletten bis blauen Halbleiter- oder Halbleiterlaserdiode ein zu untersuchender Bereich beleuchtet, und mit einer CCD-Kamera wird die Fluoreszenz beobachtet. Hierzu wird die Beleuchtungseinrichtung der oben beschriebenen Kamera mit entsprechenden UV-Leucht- bzw. Laserdioden ausgestattet, und in den Strahlengang der Optik ein Farbglasfllter mit Kantencharakteristik (z.B. GG495 von Schott) eingefügt, welches das Anregungslicht vollständig absorbiert und das Fluoreszenzlicht zum CCD-Bildwandler durchläßt. Das Umgebungslicht oder anderes Störlicht kann analog zu obigem Verfahren durch Zeittaktung von Anregungslicht und gleichzeitige sowie ungleichzeitige Detektion des Fluoreszenz-und des Störlichtbildes bzw. im Spektralbereich durch Durch-schalten zweier optischer Filter zum Messen der Störlicht- und Emissionslicht-intensität erfolgen.

Auch kann eine Auswertung der Farbverschiebung bei einem Farb-CCD-Bildwandler erfolgen.

Auch kann die Fluoreszenz der Bakterien durch bestimmte Substanzen, mit welchen zuvor der Mundraum gespült worden ist, verstärkt werden. So ist z.B. aus der photodynamischen Krebstherapie bekannt, daß Aminolävulinsäure und Derivate hiervon in Krebszellen in Porphyrine umgewandelt werden, welche dort nur schwer wieder abgebaut werden. Das gleiche ist in vielen Bakterien der Fall. Also kann Mundspülung mit einer solchen Substanz das Fluoreszenzsignal erhöhen.

In dem geschilderten Anwendungsbeispiel mit einer Kamera ist die Untersuchung eines gesamten Ortsbereiches simultan möglich. So können z.B. beginnende Karieserkrankungen an Zahnoberflächen sichtbar gemacht werden.

Aber es ist auch möglich Hauterkrankungen, wie Akne, die durch optisch anregbare Bakterien verursacht wird, oder Hautmelanome, in denen die Porphyrin-Konzentration durch erhöhten Stoffwechsel gegenüber Leberflecken deutlich vergrößert ist, sichtbar zu machen.

Zur Abschottung des Meßbereiches gegen Störlicht kann man eine zylindrisch oder kegelförmig geformte Kappe auf das Eintrittsfenster der Kamera stecken.

Da die durch die Lichtintensitäten ausgelösten Ströme im CCD-Bildwandler gegenüber dem Dunkelstrom des Bildwandlers gering sein können, ist für einen sehr empfindlichen Nachweis der CCD-Bildwandler z.B. mit einem Peltierelement zu kühlen.

Möchte man in einem schwer optisch zugänglichen Spalt, wie z.B. den Nasennebenhöhlen, dem Ohr, oder dem Zahnwurzelspalt, eine Erkrankung diagnostizieren, ist der Bereich entweder mit einem Endoskop, welches eine oben beschrieben Videokamera mit Beleuchtungsquelle enthält, zu beleuchten und abzufilmen oder man beschränkt sich auf die lokale Anwendung mit einem nicht bildgebenden System.

Dieses kann so ausgelegt sein, daß das LED- oder Laserlicht durch eine Lichtleitfaser zu dem zu untersuchenden Bereich geleitet wird. Parallel zu dieser Faser kann eine zweite Faser verlaufen, welche die von dem Bereich emittierte Fluoreszenz aufnimmt und an einen Photodetektor weiterleitet.

Weiterhin kann es im Falle von Messungen im Wurzelspalt z.B. zweckmäßig sein, den zu untersuchenden Bereich von Verschmutzungen wie Blut freizuhalten, um einen optischen Zugang zum Untersuchungsfeld zu haben. In diesem Fall kann eine weitere Hohlfaser angewendet werden, durch welche eine Spülflüssigkeit zu dem zu untersuchenden Bereich gepumpt wird.

Eine weitere Ausgestaltung der Erfindung besteht darin, mit nur einer Faser sowohl das Anregungslicht zuzuführen als auch das Fluoreszenzlicht abzunehmen. In diesem Fall ist auf der Einkoppelseite des Laserlichtes bzw. Detektionsseite des Fluoreszenzlichtes ein Strahlteiler vorzusehen, der z.B. wellenlängendispersiv beschichtet sein kann, so daß das blaue Laserlicht transmittiert, das Fluoreszenzlicht jedoch um 90° reflektiert wird.

Auch ist es möglich, die Faser teilweise als Hohlfaser auszulegen, so daß die Spülflüssigkeit durch die Faser zu der zu untersuchenden Oberfläche geleitet wird. In diesem Fall muß in dem Laser zugewandten Endbereich die Flüssigkeit durch eine seitliche Bohrung eingespeist werden.

Die oben aufgeführten erfindungsgemäßen Diagnostik-Kameras können auch angewendet werden, wenn die Bakterien durch einen zusätzlich applizierten Fluoreszenzmarker markiert worden sind. Solche Marker werden vorzugsweise vor der Untersuchung in flüssiger Lösung dem zu untersuchenden Bereich zugeführt und reichern sich spezifisch an den zu untersuchenden Bakterien an. Im Falle eines FaserBildwandlers kann der Fluoreszenzmarker vor der Untersuchung durch die Spülfaser zugeführt werden.

Bei hohem Anreicherungsgrad des Fluoreszenzmarkers in dem Untersuchungsgebiet ist es weiterhin möglich, nicht die Fluoreszenz nachzuweisen, sondern die Absorption des Fluoreszenzmarkers in dem Untersuchungsgebiet. Diese Nachweismethode ist jedoch i.a. unempfindlicller als der Fluoreszenznachweis. Apparativ ist er möglich, indem die optischen Komponenten für farbliche Trennung des Emissionssignals in dem Diagnostikgerät entfernt werden.

Mittels dieser Methode wäre zum Beispiel auch eine Detektion des verfärbten Zahnsteines auf der Zahnwurzel in der Zahntasche möglich. Dies Verfahren ist z.B. zur Analyse des Reinigungsgrades der Zahnwurzel von Zahnstein bei einer Vektorbehandlung angebracht.

Da das absolute Fluoreszenzsignal u.a. von der Ankoppeleffizienz der Optik, dem Abstand der Beleuchtung und der Oberflächenrauhigkeit sowie an die Region angrenzenden Restauratonsmaterialien abhängt, ist der Erkrankungsgrad oft nicht ohne weiteres aus der absoluten Fluoreszenzintensität feststellbar.

In diesem Fall, bietet sich eine weitere Ausgestaltung der Erfindung an. Dazu wird die Eigenschaft ausgenützt, daß bei UV-Anregung gesundes Zahnmaterial ebenfalls fluoresziert, aber mit einem anderen Emissionsspektrum. Tritt ein Erkrankungszustand ein, verändert sich das Emissionsspektrum dadurch, daß ein Teil des Anregungslichtes in den Bakterien bzw. befallenen Zellen in ein Emissionslicht anderer Wellenlänge umgewandelt wird. Diese Veränderung des Spektrums gegenüber dem gesunden Zustand kann mit einem farbempfindlichen Bildwandler in Form einer Farbveränderung erkannt werden. Diese Farbveränderung kann z.B. mit Farbkameras ortsaufgelöst erfasst und auf einem Monitor zur Anzeige des Krankheitszustands wiedergegeben werden. Damit ist nicht mehr die absolute Intensität für die Diagnose relevant.

Generell, kann also erfindungsgemäß das Verhältnis der Intensitäten verschiedener Spektralanteile verwendet werden, um ein Maß für die Erkrankung zu erhalten. Dabei repräsentiert der eine Spektralanteil das von der Erkrankung nicht beeinflußte Referenzsignal und der andere Spektralanteil das vorwiegend durch die Erkrankung beeinflußte Signal.

## Patentansprüche

1. Medizinische Kamera, mit
a) einer als ultraviolette bis blaue Halbleiter- oder Halbleiterlaserdiode ausgestalteten UV-Lichtquelle (55);
b) einem Bildwandler (8);
c) einer Optik (22, 28, 34; 80) zum Abbilden eines Untersuchungsbereichs auf den Bildwandler (8);
d) einem Filter (16; 59, 80);
e) einem Gehäuse (10);
f) einem Eintrittsfenster (16);
wobei
g) die UV-Lichtquelle (55), die Optik (22, 28, 34; 80) und der Bildwandler (8) in dem Gehäuse (10) angeordnet sind;
h) das Eintrittsfenster (16) vom Gehäuse (10) getragen ist und nicht direkt von von der UV-Lichtquelle abgegebenem Licht erreicht werden kann; und
i) das Filter durch das Eintrittsfenster (16) gebildet ist oder im Strahlengang zwischen dem Eintrittsfenster (16) und dem Bildwandler (8) angeordnet ist.

2. Kamera nach Anspruch 1, **dadurch gekennzeichnet, dass** das Filter (16; 59; 84) ein Kantenfilter ist.

3. Kamera nach Anspruch 2, **dadurch gekennzeichnet, dass** die Filterkante bei einer Wellenlänge von 450 nm oder mehr liegt.

4. Kamera nach Anspruch 3, **dadurch gekennzeichnet, dass** die Filterkante des Kantenfilters bei 470 nm oder mehr, vorzugsweise bei 550 nm oder mehr liegt.

5. Kamera nach einem der Ansprüche 1 bis 4, **dadurch gekennzeichnet, dass** die UV-Lichtquelle mindestens eine UV-LED (55) umfasst.

6. Kamera nach Anspruch 5, **dadurch gekennzeichnet, dass** die UV-Lichtquelle einen Satz von UV-LEDs (55) umfasst, die regelmäßig verteilt das Eintrittsfenster (16) umgeben.

7. Kamera nach einem der Ansprüche 1 bis 6, **dadurch gekennzeichnet, dass** die UV-Lichtquelle (55) getaktet betrieben wird.

8. Kamera nach Anspruch 7, **dadurch gekennzeichnet, dass** die Dauer und/oder der Abstand der Taktimpulse einstellbar ist.

9. Kamera nach einem der Ansprüche 1 bis 8, **dadurch gekennzeichnet, dass** zusätzlich eine Weißlichtquelle (64) vorgesehen ist.

10. Kamera nach Anspruch 9, **dadurch gekennzeichnet, dass** die Weißlichtquelle eine Mehrzahl von Weißlicht-LEDs (64) umfasst, welche regelmäßig um das Eintrittsfenster (16) herum verteilt sind und vorzugsweise getaktet betrieben werden.

11. Kamera nach einem der Ansprüche 7 bis 10, **dadurch gekennzeichnet, dass** ein Taktgeber (72) neben ersten Aktivierungsimpulsen, die zur Ansteuerung der UV-Lichtquelle (55) dienen, Steuerimpulse bereitstellt, welche zu den ersten Taktimpulsen phasenverschoben sind und dass die ersten Taktimpulse und die Steuerimpulse auf einen Rechenkreis (74) gegeben werden, der mit einem Integrations-Bildspeicher (76) zusammenarbeitet, wobei der Rechenkreis (74) bei Erhalt eines ersten Taktsignals das Ausgangssignal des Bildwandlers (8) zum Inhalt des Bildspeichers (76) hinzu addiert, während er bei Erhalt eines Steuerimpulses das vom Bildwandler (8) bereitgestellte Bild vom im Bildspeicher (76) gehaltenen integrierten Bild abzieht.

12. Kamera nach einem der Ansprüche 1 bis 11, **dadurch gekennzeichnet, dass** sie mehrere UV-Lichtquellen (55, 65) umfasst, die mit unterschiedlicher Wellenlänge arbeiten.

13. Kamera nach Anspruch 12, **dadurch gekennzeichnet, dass** ein Taktgeber (72) erste Taktimpulse zur Aktivierung der ersten UV-Lichtquelle (55) und zweite Taktimpulse zur Aktivierung der zweiten UV-Lichtquelle (64) bereitstellt, die in der Phasenlage zu den ersten Taktimpulsen verschoben sind, und Steuerimpulse bereitstellt, die zu beiden Taktimpulsen in der Phase und ein Rechenkreis (74) mit beiden Taktimpulsen beaufschlagt ist, welcher mit einem Integrations-Bildspeicher (76) zusammenarbeitet und bei Erhalt eines ersten Taktimpulses bzw. eines zweiten Taktimpulses den Inhalt des Bildwandlers (8) zum Inhalt des Bildspeichers (76) addiert oder abzieht und bei Erhalt eines Steuerimpulses den Inhalt des Bildwandlers (8) zum Inhalt des Bildspeichers (76) von diesem abzieht.

14. Kamera nach einem der Ansprüche 1 bis 13, **dadurch gekennzeichnet, dass** die Optik (80) eine Faseroptik umfasst.

15. Kamera nach einem der Ansprüche 1 bis 14, **gekennzeichnet durch** eine Einrichtung (86) zum Zuführen eines Behandlungs- oder Spülfluids zu dem zu untersuchenden Bereich.

16. Kamera nach einem der Ansprüche 1 bis 15, **dadurch gekennzeichnet, dass** eine das Bildfeld bestimmende Blende (B*) vorgesehen ist und der Bildwandler (8) auf der Achse der Optik (4) angeordnet ist, wobei die Optik (4) eine objektseitige Linsenanordnung (22), eine mittlere Linsenanordnung (28) und eine wandlerseitige Linsenanordnung (34) aufweist und dass die das Gesichtsfeld bestimmende Blende (B*) oder ein Bild (B) derselben im Bereich der wandlerseitigen Linsenanordnung (34) liegt.

17. Kamera nach Anspruch 16, **dadurch gekennzeichnet, dass** die das Gesichtsfeld bestimmende Blende (B*) oder ihr Bild (B) um eine kleine Strecke hinter der wandlerseitigen Linsenanordnung (34) liegt, welche etwa 2 bis etwa 10 %, vorzugsweise etwa 2 bis etwa 5 % des Abstandes zwischen der hinteren Begrenzungsfläche (38) der wandlerseitigen Linsenanordnung (34) und der lichtempfindlichen Fläche des Bildwandlers (8) beträgt.

18. Kamera nach Anspruch 16 oder 17, **dadurch gekennzeichnet, dass** die objektseitige Linsenanordnung (22) durch eine konkav/konvex gekrümmte Linse (22) gebildet ist.

19. Kamera nach einem der Ansprüche 16 bis 18, **dadurch gekennzeichnet, dass** die mittlere Linsenanordnung (28) durch eine bikonvexe Linse gebildet ist.

20. Kamera nach einem der Ansprüche 16 bis 19, **dadurch gekennzeichnet, dass** die wandlerseitige Linsenanordnung (34) durch eine bikonvexe Linse gebildet ist.

21. Kamera nach einem der Ansprüche 16 bis 20, **gekennzeichnet, durch** ein vor der objektseitigen Linsenanordnung (21) angeordnetes Lichtumlenkmittel (18).

22. Kamera nach einem der Ansprüche 16 bis 21, **dadurch gekennzeichnet, dass** ein vor der objektseitigen Linsenanordnung (22) liegendes Eintrittsfenster (16) bündig und dicht mit dem Gehäuse (10) verbunden ist.

23. Kamera nach einem der Ansprüche 16 bis 22, **gekennzeichnet durch** eine Einrichtung (46, 48; 60, 62) zum Verstellen des Bildwandlers (8) in Richtung der Achse der Optik (4).

24. Kamera nach Anspruch 23, **dadurch gekennzeichnet, dass** die Verstelleinrichtung ein durch eine Wand des Gehäuses (10) hindurchgeführtes Betätigungselement (48) aufweist.

25. Kamera nach Anspruch 23, **dadurch gekennzeichnet, dass** die Verstelleinrichtung einen Elektromotor (62) aufweist, der über eine Steckverbindung erregt wird, über welche auch der Bildwandler (8) mit einer Bildauswerteelektronik verbunden ist.

26. Kamera nach einem der Ansprüche 1 bis 25, **dadurch gekennzeichnet, dass** der Untersuchungsbereich durch eine zylindrisch oder kegelförmig geformte Kappe gegen Störlicht abgeschottet ist, welches auf das Eintrittsfenster (16) gesteckt ist.

27. Kamera nach einem der Ansprüche 1 bis 26, **dadurch gekennzeichnet, dass** die Kamera einen farbempfindlichen Bildwandler aufweist, so dass, wenn ein Teil des Anregungslichts in den Bakterien bzw. befallenen Zellen in ein Emissionslicht anderer Wellenlänge umgewandelt wird, diese Veränderung des Spektrums gegenüber dem gesunden Zustand mit dem farbempfindlichen Bildwandler in Form einer Farbveränderung erkannt werden kann.

28. Kamera nach Anspruch 27, **dadurch gekennzeichnet, dass** die Kamera eine Bildauswerteelektronik umfasst, welche das Verhältnis der Intensitäten verschiedener Spektralanteile verwendet, um ein Maß für die Erkrankung zu erhalten.

## Claims

1. A medical camera comprising,
a) a UV-light source (55) configured as an ultraviolet to blue semiconductor or semiconductor laser diode;
b) an image converter (8);
c) an optical system (22, 28, 34; 80) adapted for imaging a region to be diagnosed on the image converter (8);
d) a filter (16; 59, 80);
e) a housing (10);
f) an entrance window (16),
wherein
g) the UV-light source (55), the optical system (22, 28, 34; 80) and the image converter (8) are arranged in the housing (10);
h) the entrance window (16) is carried by the housing (10) and is not accessible directly by light emitted from the UV-light source (55); and wherein
i) the filter is formed by the entrance window (16) or is arranged in the optical path defined between the entrance window (16) and the image converter (8).

2. The medical camera according to claim 1, **characterized in that** the filter (16; 59, 84) is an edge filter.

3. The medical camera according to claim 2, **characterized in that** the filter edge is at a wavelength of 450 nm or more.

4. The medical camera according to claim 3, **characterized in that** the filter edge of the edge filter is at 470 nm or more, preferably at 550 nm or more.

5. The medical camera according to one of the claims 1 to 4, **characterized in that** the UV-light source comprises at least one UV-LED (55).

6. The medical camera according to claim 5, **characterized in that** the UV-light source comprises a set of UV-LEDs (55) surrounding the entrance window (16) under equal pitch.

7. The medical camera according to one of the claims 1 to 6, **characterized in that** the UV-light source is intermittently energized.

8. The medical camera according to claim 7, **characterized in that** the length and/or the distance of the intermittent pulses is adjustable.

9. The medical camera according to one of the claims 1 to 8, **characterized in that** a white light source (64) is additionally provided.

10. The medical camera according to claim 9, **characterized in that** the white light source comprises a plurality of white light LEDs (64) arranged around the entrance window (16) under regular pitch and preferably energized intermittently.

11. The medical camera according to one of the claims 7 to 10, **characterized in that** a timer (72) in addition to first activating pulses for control of the UV-light source (55) provides control pulses being phase shifted with respect to the first activating pulses and **in that** the first activating pulses and the control pulses are supplied to a computing circuit (74) co-operating with an integrating image memory (76), the computing circuit (74) upon receipt of the first activating pulses adding the signal output from the image converter (8) to the contents of the image memory (76) and upon receipt of a control pulse subtracting the image provided by the image converter (8) from the integrated image contained in the image memory (76).

12. The medical camera according to one of the claims 1 to 11, **characterized in that** it comprises a plurality of UV-light sources (55, 64) operating at different wavelengths.

13. The camera according to claim 12, **characterized in that** a timer (72) provides first timing pulses for activating the first UV-light source (55) and second timing pulses to activate the second UV-light source (64), the second timing pulses being phase shifted with respect to the first timing pulses, and **in that** the timer also provides control pulses, which are in phase to the first and second timing pulses, a computing circuit (74) being provided for receiving the two timing pulses and co-operating with an integrating image memory (76) such that upon receipt of a first timing pulse the contents of the image converter (8) is added to the contents of the image memory (76), respectively upon receipt of a second timing pulse the contents of the image converter (8) is subtracted from the image memory (76) and that upon receipt of a control pulse the contents of the image converter (8) is subtracted from the contents of the image memory (76).

14. The camera according to one of the claims 1 to 13, **characterized in that** the optical system (80) comprises a fiber optic system.

15. The camera according to one of the claims 1 to 14, **characterized by** a means (86) for supplying a treatment fluid or a rinsing fluid to the region to be diagnosed.

16. The camera according to one of the claims 1 to 15, **characterized in that** a diaphragm (B*) defining the field of view is provided and **in that** the image converter (8) is arranged on the axis of the optical system (4), the optical system (4) comprising a lens arrangement being close to the object (22), an intermediate lens arrangement (28) and a lens arrangement (34) being close to the image converter (8) and **in that** the diaphragm (B*) defining the field of view or an image (B) thereof is arranged in a region of the lens arrangement (34) being close to the image converter.

17. The camera according to claim 16, **characterized in that** the diaphragm (B*) defining the field of view or an image (B) thereof is situated a small distance behind the lens arrangement (34) being close to the image converter, which distance is about 2 to about 10%, preferably 2 to about 5% of the distance defined between the rear end face (38) of the lens arrangement (34) being close to the image converter and the light sensitive surface of the image converter (8).

18. The camera according to claim 16 or claim 17, **characterized in that** the lens arrangement (22) being close to the object is formed by a lens being concavely/convexly curved.

19. The camera according to one of the claims 16 to 18, **characterized in that** the intermediate lens arrangement (28) is formed by a bi-convex lens.

20. The camera according to one of the claims 16 to 19, **characterized in that** the lens arrangement (34) being close to the image converter is formed by a bi-convex lens.

21. The camera according to one of the claims 16 to 20, **characterized in that** a light deflecting means (18) is arranged in front of the lens arrangement (21) being close to the object.

22. The camera according to one of the claims 16 to 21, **characterized in that** an entrance window (16) arranged in front of the lens arrangement (22) being close to the object is connected to the housing (10) in a flush and a fluid tight manner.

23. The camera according to one of the claims 16 to 22, **characterized by** a means for positioning the image converter (8) along the axis of the optical system.

24. The camera according to claim 23, **characterized in that** the positioning means comprises an actuating element (48) extending through a wall of the housing (10).

25. The camera according to claim 23, **characterized in that** the positioning means comprises an electric motor (62) which is energized by a connector means which also provides an electric connection between the image converter (8) and an electronic image evaluating unit.

26. The camera according to one of the claims 1 to 25, **characterized in that** the region to be diagnosed is protected against ambient light by means of a cylindrical or conical cap member arranged on the entrance window (16).

27. The camera according to one of the claims 1 to 26, **characterized in that** the camera comprises a color sensitive image converter, so that when a part of the stimulating light is transformed into probe light of different wavelength by bacteria or ill cells, this transformation in the spectrum with respect to a healthy state is detected using the color sensitive image converter as a change of color.

28. The camera according to claim 27, **characterized in that** the camera comprises an electronic image evaluating unit, which uses the ratio between intensities of different spectral components to obtain a measure for the illness.

## Revendications

1. Caméra médicale, dotée
a) d'une source de lumière UV (55) conçue sous la forme d'une diode à semi-conducteur ou d'une diode laser à semi-conducteur de rayonnement ultraviolet à bleu ;
b) d'un convertisseur d'images (8) ;
c) d'une optique (22, 28, 34 ; 80) destinée à reproduire en image une zone faisant l'objet d'une analyse sur le convertisseur d'images (8) ;
d) d'un filtre (16 ; 59, 80) ;
e) d'un boîtier (10);
f) d'une fenêtre d'entrée (16) ;
dans laquelle
g) la source de lumière UV (55), l'optique (22, 28, 34 ; 80) et le convertisseur d'images (8) sont agencés dans le boitier (10) ;
h) la fenêtre d'entrée (16) est ménagée dans le boîtier (10) et ne peut pas être directement atteinte par la lumière délivrée par la source de lumière UV ; et
i) le filtre est formé à travers la fenêtre d'entrée (16) ou est agencé dans la trajectoire du faisceau entre la fenêtre d'entrée (16) et le convertisseur d'images (8).

2. Caméra selon la revendication 1, **caractérisée en ce que** le filtre (16 ; 59 ; 84) est un filtre de coupure.

3. Caméra selon la revendication 2, **caractérisée en ce que** la bande passante du filtre commence à une longueur d'onde de 450 nm ou plus.

4. Caméra selon la revendication 3, **caractérisée en ce que** la bande passante du filtre de coupure commence à une longueur d'onde de 470 nm ou plus, de préférence à une longueur d'onde de 550 nm ou plus.

5. Caméra selon l'une des revendications 1 à 4, **caractérisée en ce que** la source de lumière UV comprend au moins une LED UV (55).

6. Caméra selon la revendication 5, **caractérisée en ce que** la source de lumière UV comprend un ensemble de LED UV (55), qui entourent la fenêtre d'entrée (16) en étant réparties de manière régulière.

7. Caméra selon l'une des revendications 1 à 6, **caractérisée en ce que** la source de lumière UV (55) fonctionne en synchronisation.

8. Caméra selon la revendication 7, **caractérisée en ce que** la durée et/ou la période de l'impulsion de synchronisation est réglable.

9. Caméra selon l'une des revendications 1 à 8, **caractérisée en ce qu'**une source de lumière blanche (64) est en outre prévue.

10. Caméra selon la revendication 9, **caractérisée en ce que** la source de lumière blanche comprend une pluralité de LED de lumière blanche (64), lesquelles sont réparties de manière régulière autour la fenêtre d'entrée (16) et fonctionnent de préférence en synchronisation.

11. Caméra selon l'une des revendications 7 à 10, **caractérisée en ce qu'**une horloge (72) fournit en plus des premières impulsions d'activation, qui sont destinées à exciter la source de lumière UV (55), des impulsions de commande, lesquelles sont déphasées par rapport aux premières impulsions de synchronisation et **en ce que** les premières impulsions de synchronisation et les impulsions de commande sont délivrées à un circuit de calcul (74), qui fonctionne conjointement avec une mémoire image d'intégration (76), où le circuit de calcul (74) ajoute à réception d'un premier signal de synchronisation le signal de sortie du convertisseur d'images (8) au contenu de la mémoire image (76), tandis qu'il soustrait à réception d'une impulsion de commande l'image fournie par le convertisseur d'images (8) de l'image intégrée contenue dans la mémoire image (76).

12. Caméra selon l'une des revendications 1 à 11, **caractérisée en ce qu'**elle comprend plusieurs sources de lumière UV (55, 65), qui fonctionnent à une longueur d'onde différente.

13. Caméra selon la revendication 12, **caractérisée en ce qu'**une horloge (72) fournit des premières impulsions de synchronisation destinées à activer la première source de lumière UV (55) et des deuxièmes impulsions de synchronisation destinées à activer la deuxième source de lumière UV (64), qui présentent un décalage de phase par rapport aux premières impulsions de synchronisation, et fournit une impulsion de commande, qui est délivrée sans décalage de phase par rapport aux deux impulsions et un circuit de calcul (74) avec les deux impulsions de synchronisation, lequel fonctionne conjointement avec une mémoire image d'intégration (76) et ajoute ou soustrait à réception d'une première impulsion de synchronisation ou d'une deuxième impulsion de synchronisation le contenu du convertisseur d'images (8) au contenu de la mémoire image (76) et soustrait à réception d'une impulsion de commande le contenu du convertisseur d'images (8) du contenu de la mémoire image (76).

14. Caméra selon l'une des revendications 1 à 13, **caractérisée en ce que** l'optique (80) comprend une optique à fibres de verre.

15. Caméra selon l'une des revendications 1 à 14, **caractérisée par** un dispositif (86) destiné à acheminer un fluide de traitement ou de rinçage jusqu'à la zone analysée.

16. Caméra selon l'une des revendications 1 à 15, **caractérisée en ce qu'**un diaphragme (B*) positionné dans le champ d'image est prévu et le convertisseur d'images (8) est agencé sur l'axe de l'optique (4), où l'optique (4) présente un agencement de lentille côté objet (22), un agencement de lentille central (28) et un agencement de lentille côté convertisseur (34) et **en ce que** le diaphragme (B*) positionné dans le champ optique ou une image (B) de celui-ci se situe dans la zone de l'agencement de lentille côté convertisseur (34).

17. Caméra selon la revendication 16, **caractérisée en ce que** le diaphragme (B*) positionné dans le champ optique ou son image (B) se situe à une petite distance derrière l'agencement de lentille côté convertisseur (34), laquelle distance est comprise entre environ 2 et environ 10 %, de préférence entre environ 2 et environ 5 %, de l'écart entre la périphérie arrière (38) de l'agencement de lentille côté convertisseur (34) et la surface sensible à la lumière du convertisseur d'images (8).

18. Caméra selon la revendication 16 ou 17, **caractérisée en ce que** l'agencement de lentille côté objet (22) se présente sous la forme d'une lentille courbe concave/convexe (22).

19. Caméra selon l'une des revendications 16 à 18, **caractérisée en ce que** l'agencement de lentille central (28) se présente sous la forme d'une lentille biconvexe.

20. Caméra selon l'une des revendications 16 à 19, **caractérisée en ce que** l'agencement de lentille côté convertisseur (34) se présente sous la forme d'une lentille biconvexe.

21. Caméra selon l'une des revendications 16 à 20, **caractérisée par** un moyen de déviation de lumière (18) agencé devant l'agencement de lentille côté objet (21).

22. Caméra selon l'une des revendications 16 à 21, **caractérisée en ce qu'**une fenêtre d'entrée (16) située devant l'agencement de lentille côté objet (22) est en affleurement par rapport au boîtier (10) et est incorporée dans l'épaisseur de celui-ci.

23. Caméra selon l'une des revendications 16 à 22, **caractérisée par** un dispositif (46, 48 ; 60, 62) destiné à déplacer le convertisseur d'images (8) selon la direction de l'axe de l'optique (4).

24. Caméra selon la revendication 23, **caractérisée en ce que** la direction de déplacement présente un élément d'actionnement (48) introduit dans une paroi du boîtier (10).

25. Caméra selon la revendication 23, **caractérisée en ce que** la direction de déplacement présente un moteur électrique (62), qui est excité par l'intermédiaire d'une fiche de raccordement, à laquelle le convertisseur d'images (8) doté d'une électronique de restitution de prises de vues est également raccordé.

26. Caméra selon l'une des revendications 1 à 25, **caractérisée en ce que** la zone faisant l'objet d'une analyse est encerclée par un capuchon moulé cylindrique ou conique afin de la protéger de la lumière parasite, laquelle s'introduit dans la fenêtre d'entrée (16).

27. Caméra selon l'une des revendications 1 à 26, **caractérisée en ce que** la caméra présente un convertisseur d'images sensible aux couleurs, de telle sorte que, lorsqu'une partie de la lumière d'excitation dans les cellules bactériennes ou contaminées est convertie en une lumière d'émission d'une autre longueur d'onde, cette transformation du spectre par rapport à l'état sain avec le convertisseur d'images sensible aux couleurs peut être visualisée sous la forme d'une transformation de couleur.

28. Caméra selon la revendication 27, **caractérisée en ce que** la caméra comprend une électronique de restitution des prises de vues, laquelle utilise le rapport de l'intensité de la partie spectrale différente, afin d'obtenir une mesure pour la maladie.
